# EUROPÄISCHE PATENTSCHRIFT

(11) **EP 0 842 142 B1**
(45) Veröffentlichungstag und Bekanntmachung des Hinweises auf die Patenterteilung: **26.09.2001**
(21) Anmeldenummer: 96925746.8
(22) Anmeldetag: 12.07.1996
(51) Int. Cl.: C07C 231/02, C07K 1/08, C07K 1/06

(54) **VERFAHREN ZUR HERSTELLUNG VON ALPHA-(N,N-DIALKYL)-AMINOCARBONSÄUREAMIDEN**
PROCESS FOR PRODUCING ALPHA-(N,N DIALKYL)-AMINO CARBOXYLIC ACID AMIDES
PROCEDE DE PREPARATION DE ALPHA-(N,N-DIALKYLE)-AMIDES D'ACIDE AMINOCARBOXYLIQUE

(30) Priorität: 28.07.1995 DE 19527574
(43) Veröffentlichungstag der Anmeldung: 20.05.1998
(73) Patentinhaber: BASF AKTIENGESELLSCHAFT, 67056 Ludwigshafen (DE)
(72) Erfinder: BUSCHMANN, Ernst, D-67069 Ludwigshafen (DE); ZIERKE, Thomas, D-67459 Böhl-Iggelheim (DE)
(86) Internationale Anmeldenummer: EP9603075
(87) Internationale Veröffentlichungsnummer: WO9705096

(56) Entgegenhaltungen:
- EP-A- 0 014 834
- JOURNAL OF THE AMERICAN CHEMICAL SOCIETY, Bd. 113, 1991, DC US, Seiten 6692-6693, XP002020002 G.R. PETTIT ET AL. : "Antineoplastic Agents. 220. Synthesis of Natural Dolastatin" in der Anmeldung erwähnt

## Beschreibung

Die Erfindung betrifft ein neues Verfahren zur racemisierungsfreien Verknüpfung von a-(N,N-Dialkyl)-aminosäuren mit Aminen zu den entsprechenden Carbonsäureamiden.

Bei der Synthese zahlreicher Naturstoffe mit interessantem pharmakologischem Wirkprofil und bei den von diesen Substanzen strukturell abgeleiteten Wirkstoffen sind α-(N,N-Dialkyl)-aminosäuren der Formel II 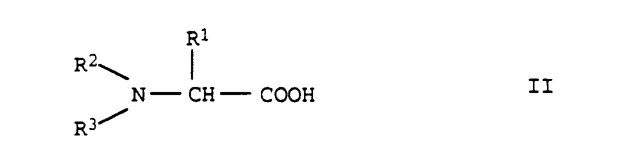 als Bausteine von Interesse (z.B. Enkephaline: U. Schmidt et al.; Liebigs Ann. Chem. 1985, 1254-1262, Dolastatine: G.R. Pettit et al.; Tetrahedron 49 (41) 9151 - 9170, WO 93/23 424). So wird beispielsweise aus N,N-Dimethylvalin (IIa) und einem Tetrapeptid der antineoplastische Wirkstoff Dolastatin 10 hergestellt:

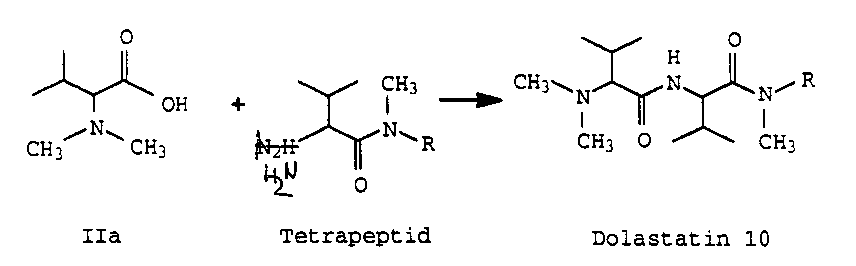

Die Verknüpfung einer N,N-Dialkylaminosäure wie IIa zu Peptiden ist schwierig und gelang im Fall des Dolastatins 10 nur mit einer Ausbeute von 53 % (J. Poncet et al. Tetrahedron 50, 5345-5360 (1994)). Dieses schlechte Ergebnis, durch das ein großer Teil des wertvollen Tetrapeptides bei der Dolastatin-Synthese verloren geht, ist nach U. Schmidt et al. (Liebigs Ann. 1985, 1254-1262) nicht überraschend. U. Schmidt hat auch die Verknüpfung von N,N-Dimethylaminosäuren im Zusammenhang mit der Synthese von Enkephalinen untersucht. Zur Aktivierung dieser Aminosäuren erwiesen sich dabei nur drei Methoden als brauchbar:
1. Die Aktivierung der N,N-Dimethylaminosäure durch Überführen in die Pentafluorphenylester: 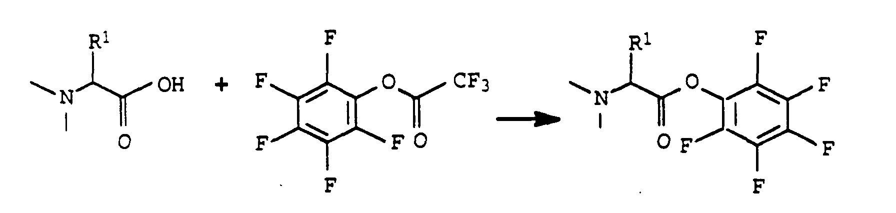
   Diese Methode wird beispielsweise von G.R. Pettit et al. (J.Am.Chem.Soc. 1991, 113 (6692-6693) für die Synthese von Dolastatin 15 verwendet. Als Ausgangsverbindung für dieses mehrstufige Verfahren benötigt man Pentafluorphenol, ein teures Reagenz, das für technische Synthesen nicht in genügender Menge zur Verfügung steht. Dessen Verwendung führt darüber hinaus zu fluorhaltigen Abfällen, die nur schwer und möglicherweise unter Dioxinbildung zu entsorgen sind.
2. Als Alternative empfiehlt U. Schmidt die Aktivierung mit 3-Cyan-4,6-dimethyl-2-pyridinthiol: 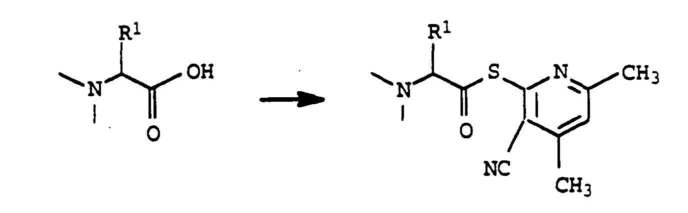
   Das Cyanopyridin ist jedoch kommerziell nicht erhältlich und muß in einem mehrstufigen Verfahren hergestellt werden.
3. Eine dritte Alternative ist die Aktivierung der Dimethylaminosäure mit Diethylphosphorcyanidat (= DEPC) (G.R. Pettit, Tetrahedron 1994 50 (42, 12097-12108). Auch dieses Reagenz ist kommerziell nicht in den Mengen erhältlich, die für eine technische Synthese notwendig sind. Ein zusätzlicher mehrstufiger Prozeß ist erforderlich. Bei der Herstellung und Umsetzung von DEPC muß mit hochgiftigen cyanidhaltigen Lösungen gearbeitet werden.

Dringend benötigt werden deshalb Verfahren, die eine einfache Verknüpfung von N,N-Dimethylaminosäuren ermöglichen.

Interesse besteht jedoch nicht nur an der peptidischen Verknüpfung von N,N-Dimethylaminosäuren, sondern auch an Peptidkopplungen mit N-Benzyl-N-methylaminosäuren. Der Benzylrest dient dabei als Schutzgruppe, die leicht hydrogenolytisch abgespalten werden kann.

N-Benzyl-N-methylaminosäuren sind zwar als Peptidbausteine gut herstellbar, werden jedoch wenig verwendet, weil es bisher keine brauchbaren Methoden zur racemisierungsfreien Verknüpfung dieser Aminosäuren gibt.

Zur Herstellung von Peptiden, die zum Teil aus N-Methylaminosäuren aufgebaut sind (z.B. Cyclosporine, Dolastatine), werden deshalb ausschließlich N-Acyl-N-methylaminosäuren eingesetzt (Acyl = Butoxycarbonyl (BOC), Benzyloxycarbonyl (Z)), die zwar schwieriger herstellbar sind als N-Benzyl-N-methylaminosäuren, dafür aber bei der Verknüpfung weniger zur Racemisierung neigen (Beispiel: Dolestatin 15-Synthese von G.R. Pettit, J. Am. Chem. Soc. 1991 113 6692-6693).

Auch für die Verwendung von N-Benzyl-N-methylaminosäuren wäre daher ein geeignetes Kopplungsreagenz wünschenswert.

Die Verknüpfung von Carbonsäuren mit Aminen in Gegenwart von Anhydriden der Alkanphosphonsäuren zu den entsprechenden Amiden ist ein gebräuchliches Verfahren (EP 14.834), das auch zur Herstellung von Peptiden eingesetzt werden kann (EP 156.280). Bevorzugt wird dabei n-Propanphosphonsäureanhydrid (PPA) verwendet. Die Anwendbarkeit dieses Reagenzes wurde jedoch nur für am Stickstoff nicht alkylierte N-Acylaminosäuren gezeigt.

Für derartige Umsetzungen stehen zahlreiche Kopplungsreagenzien zur Verfügung. Die Anwendbarkeit von Anhydriden der Alkanphosphonsäuren zur Verknüpfung von N,N-Dialkylaminosäuren der Formel I wurde bisher nicht untersucht.

Im Zusammenhang mit der oben aufgezeigten Problematik war es daher überraschend, daß Anhydride der Alkanphosphonsäuren gut geeignete Kopplungsreagenzien für die Verknüpfung von α-(N,N-Dialkyl)-aminosäuren darstellen.

Gegenstand der Erfindung ist ein Verfahren zur Herstellung von α-(N,N-Dialkylamino)-carbonsäureamiden der Formel I 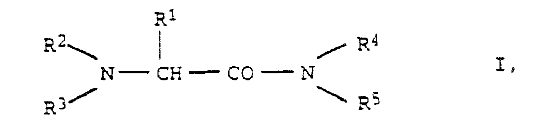 in der
- R¹: C₁₋₆-Alkyl, C₃₋₇-Cycloalkyl, Phenyl, Benzyl, (CH₂)₃NH(C=NH)NH₂, CH₂CONH₂, CH₂CO₂H, CH₂SH, (CH₂)₂CONH₂, (CH₂)₂CO₂H, Imidazolyl-5-methylen, (CH₂)₄NH₂, (CH₂)₂SCH₃, CH₂OH, CH(OH)CH₃ oder Indolyl-β-methylen bedeutet, wobei reaktive Gruppen nach Bedarf mit Schutzgruppen versehen sind,
- R²: C₁₋₆-Alkyl oder ein mit C₁₋₄-Alkyl, C₁₋₄-Alkoxy, N, CF₃, Cl, F oder Brom gegebenenfalls substituiertes Benzyl,
- R³: C₁₋₆-Alkyl oder ein mit C₁₋₄-Alkyl, C₁₋₄-Alkoxy, N, CF₃, Cl, F oder Brom gegebenenfalls substituiertes Benzyl, wobei R¹ und R³ miteinander verknüpft sein können,
- R⁴ und R⁵: unabhängig voneinander Wasserstoff, C₁₋₅-Alkyl, C₃₋₇-Cycloalkyl, Phenyl (welches durch 1, 2 oder 3 Fluor-, Chlor- oder Bromatome oder C₁₋₅-Alkyl-, C₁₋₅-Alkoxy- oder CF₃-Gruppen substituiert sein kann) einen aromatischen Heterocyclus (welcher durch 1, 2 oder 3 Fluor-, Chlor- oder Bromatome oder C₁₋₅-Alkyl-, C₁₋₅-Alkoxy- oder CF₃-Gruppen substituiert sein kann) oder Benzyl (welches durch 1, 2 oder 3 Fluor-, Chlor- oder Bromatome oder C₁₋₅-Alkyl-, C₁₋₅-Alkoxy- oder CF₃-Gruppen substituiert sein kann) bedeuten, wobei R⁴ und R⁵ noch zusätzlich miteinander verknüpft sein können, und in der NR⁴R⁵ auch für einen Aminosäure- oder einen Peptidrest stehen kann, wobei die Carboxylgruppe und andere funktionelle Gruppen eventuell mit Schutzgruppen blockiert sind,
bedeuten, dadurch gekennzeichnet, daß man die entsprechenden freien Säuren mit primären oder sekundären Aminen in Gegenwart von Anhydriden einer Alkanphosphonsäure umsetzt.

Als primäre und sekundäre Amine 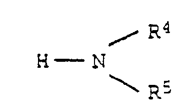 seien speziell insbesondere genannt:
- Amine wie Ammoniak, Methylamin, Ethylamin, Propylamin, Butylamin, Isobutylamin, Benzylamin, Cyclohexylamin, Dimethylamin, Diethylamin, Benzylmethylamin, Dibenzylamin, Pyrrolidon, Hydroxy- und Methylpyrrolidine, Piperidin, Hydroxy und Methylpiperidine, Anilin, N-Methylanilin, Morpholin, Alkylmorpholine, Aminopyridin;
- Aminosäurederivate wie Ester und Amide von Alanin, N-Methylalanin, Glycin, N-Methylglycin, Isoleucin, N-Methylisoleucin, Methionin, N-Methylmethionin, Phenylglycin, N-Methyl-phenylglycin, Phenylalanin, N-Methyl-phenylalanin, Prolin, Tryptophan, N-Methyltryptophan, Valin, N-Methylvalin, β-Alanin, N-Methyl-β-alanin;
- Peptide wie
   Val-MeVal-OMe, MeVal-Val-OMe, Pro-Val-OMe, Pro-Val-NH₂,
   Pro-Val-NHBz, Val-MeVal-Val-Pro-OMe, Val-MeVal-Val-Pro-NH₂,
   Val-MeVal, Val-Pro-NHBz, Val-MeVal-Val-Pro-NHiProp,
   Val-MeVal-Pro-ProNH₂, Val-MeVal-Pro-Pro-NHBz,
   Val-MeVal-Pro-Pro-NHiProp.

Ist R¹ eine C₁₋₆-Alkyl- oder eine C₃₋₇-Cycloalkylgruppe, so kommen hierfür insbesondere in Betracht: Methyl, Isopropyl, Isobutyl, 1-Methyl-n-propyl-1-yl, Cyclopropyl und Cyclohexyl.

Die für R¹ speziell genannten C₁₋₆-Alkylgruppen sind auch für R², R³, R⁴ und R⁵ bevorzugt. Als Cycloalkylgruppen seien für R⁴ und R⁵ besonders Cyclopropyl und Cyclohexyl genannt.

Als aromatische Heterocyclen kommen für R⁴ und R⁵ vorzugsweise Thiazol-, Thiophen- und Pyridin-Reste in Betracht.

R⁴ und R⁵ können auch durch folgende Brücken miteinander verbunden sein, so daß R⁴R⁵ dann für (CH₂)₄, (CH₂)₅, CH₂CH₂OCH₂CH₂, (CH₂)₆ stehen.

R¹ und R³ können miteinander verknüpft sein. Dabei werden gegebenenfalls C₁-C₄-alkylsubstituierte Pyrrolidine und Piperidine gebildet.

R³ kann neben einer Alkylgruppe für einen gegebenenfalls mit C₁-C₄-Alkyl, C₁-C₄-Alkoxy, Nitro, CF₃, Cl, F, Br substituierten Benzylrest stehen.

Das erfindungsgemäße Verfahren eignet sich also sowohl für die Herstellung von Amiden der N,N-Dialkylaminosäuren I, als auch besonders zur Herstellung von Peptiden.

Bei der Umsetzung arbeitet man bei Temperaturen von -10°C bis +40°C; vorzugsweise zwischen -5°C und Zimmertemperatur. Als Kopplungsreagenzien eignen sich die Anhydride der gerad- oder verzweigtkettigen, gegebenenfalls cyclischen Alkanphosphonsäuren mit Kettenlängen von 1 bis 8 Kohlenstoffatomen, bevorzugt das Propanphosphonsäureanhydrid (PPA). Als weitere Beispiele seien Methanphosphonsäureanhydrid, Ethanphosphonsäureanhydrid und Butanphosphonsäureanhydrid genannt.

Die Herstellung der Alkanphosphonsäureanhydride kann in an sich bekannter Weise erfolgen, wie z.B. in Houben-Weyl, Meth. d. Org. Chem. (1963), Bd. XII/1, S. 612, beschrieben.

Für die Herstellung von Peptiden arbeitet man zweckmäßig mit besonders reinen Alkanphosphonsäureanhydriden. Derartige Anhydride lassen sich gewinnen durch Umsetzung reiner Alkanphosphonsäuredichloride mit 1 Mol Wasser und anschließende Entfernung des noch im Reaktionsgut verbliebenen Chlorwasserstoffs im Vakuum.

Bevorzugt für die Herstellung dieser Anhydride ist das Verfahren der deutschen Patentanmeldung 2.811.628. Hierbei werden reine Alkanphosphonsäuren durch thermische Wasserabspaltung in die Anhydride überführt. Eine anschließende Reinigung mit Hilfe einer Vakuumdestillation kann zweckmäßig sein. Die Umsetzung erfolgt am besten in neutralem oder schwach alkalischem Medium.

Die Kondensationsreaktion wird zweckmäßig in einem gepufferten Medium vorgenommen, was durch Zugabe von aliphatischen und cycloaliphatischen tertiären Basen wie z.B. N-Methylmorpholin, N-Ethylmorpholin, Trialkylaminen mit bis zu 6 C-Atomen pro Alkylrest geschehen kann. Besonders bewährt haben sich Triethylamin und Diisopropylethylamin. Geeignete Lösungsmittel sind Dimethylsulfoxid, DMF, DMA, N-Methylpyrrolidon, Chloroform, Methylenchlorid, THF, Dioxan und Essigsäuremethylester.

Zur Herstellung von Peptiden verwendet man als Ausgangsmaterialien neben der N,N-Dialkylaminosäure bzw. der N-Benzyl-N-alkylaminosäure und dem Anhydrid der Alkanphosphonsäure eine Aminosäure oder ein Peptid mit einer blockierten Carboxylgruppe. Für den Schutz der Carboxylgruppe können alle in der Peptidsynthese üblichen Schutzgruppen verwendet werden.

Die Alkanphosphonsäureanhydride werden vorzugsweise im Überschuß (2 bis 2,5 Mol Alkanphosphonsäureanhydrid pro Mol zu knüpfender Amidbindung) eingesetzt.

Die folgenden Beispiele erläutern das erfindungsgemäße Verfahren.

### Beispiel 1

### Me₂Val-Val-MeVal-Pro-Pro-NHBz x HCl

Zu einer Lösung von 8,7 g L-N,N-Dimethylvalin, 27,4 g Val-MeVal-Pro-Pro-NHBz x HCl und 21,6 g Triethylamin in 100 ml CH₂Cl₂ wurden 42,4 g einer 50 %igen Lösung von n-Propanphosphonsäureanhydrid in Essigester bei 0 bis 65°C innerhalb von 20 min zugetropft. Man rührte 1 h in der Kälte und über Nacht bei Raumtemperatur. Die organische Phase wurde mit 50 ml Wasser gewaschen und eingeengt. Der Rückstand wurde in 50 ml Isopropanol gelöst und mit 10 ml 30%iger isopropanolischer HCl angesäuert. Man impfte an, setzte bei 60°C 150 ml Methyl-tert.-butylether zu, rührte über Nacht, saugte ab, wusch mit Isopropanol und trocknete. Man erhielt 29,9 g (88,3 %) Me₂Val-Val-MeVal-Pro-Pro-NHBz x HCl, Reinheit HPLC-Flächenprozent: 99,8 % (kein Epimer erkennbar).

### Beispiel 2

### Me₂Val-Val-MeVal-Pro-Pro-NHBz x HCl

Zu einer Lösung von 21,6 g Dimethylvalin, 54,8 g Val-MeVal-Pro-Pro-NHBz x HCl und 54 g Diisopropylethylamin in 100 ml CH₂Cl₂ wurden 84,8 g einer 50 %igen Lösung von n-Propanphosphonsäureanhydrid in Essigester bei -75°C innerhalb von 45 min zugetropft. Man rührte 1 h in der Kälte und über Nacht bei Raumtemperatur. Die organische Phase wurde mit 200 ml gesättigter NaCl-Lösung und 100 ml 10 %iger NaOH gewaschen und eingeengt. Der Rückstand wurde in 350 ml 2-Butanol gelöst. Man gab 25 ml gesättigte isopropanolische HCl-Lösung und 200 ml MTBE zu, rührte über Nacht, saugte ab und trocknete im Vakuum. Man erhielt 61,4 g (90,5 %) Produkt, Reinheit HPLC-Flächenprozent: 99,8 % (kein Epimer erkennbar).

### Beispiel 3

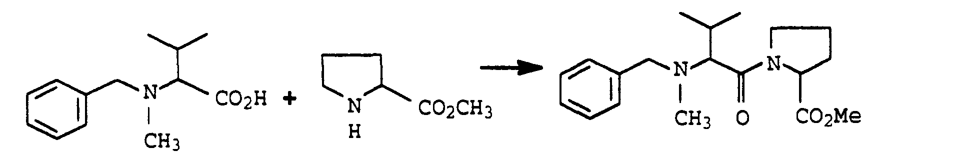

Zu einer Lösung von 50 g N-Benzyl-N-methylvalin in CH₂Cl₂ wurden 120 g Diisopropylethylamin zugegeben. Bei -55 bis 5°C wurden 37,7 g Prolinmethylester-Hydrochlorid und 176,8 g 50 %ige Lösung von Propanphosphonsäureanhydrid in Essigester zugegeben. Man rührte 1 h in der Kälte und über Nacht bei Raumtemperatur. Die organische Phase wurde mit 400 ml Wasser, 200 ml 1 N NaOH und zweimal mit Wasser gewaschen, über Na₂SO₄ getrocknet und eingeengt. Man erhielt 49,6 g (65,4 %) Produkt, Reinheit HPLC-Flächenprozent: 93,7 %. Der Gehalt an anderen Diastereomeren lag unter 1 %.

### Beispiel 4

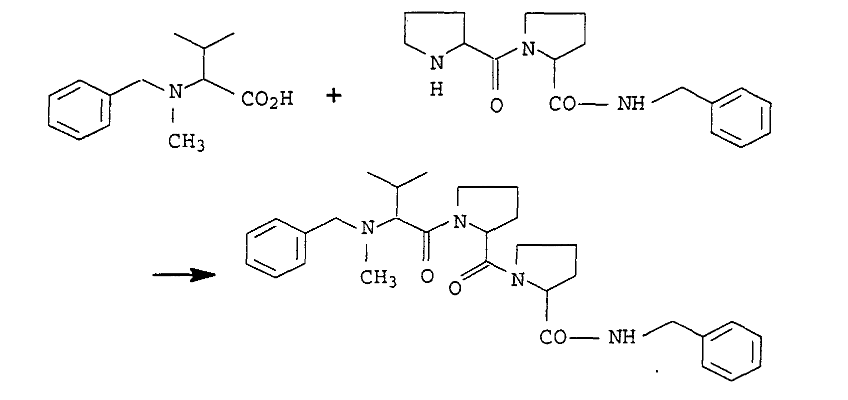

Zu einer Lösung von 6,5 g N-Benzyl-N-methyl-valin und 16,3 g Diisopropylethylamin in Methylenchlorid wurden bei -55°C bis 5°C 9,9 g Pro-Pro-NHBz x HCl und 24,3 g einer 50 %igen Lösung von n-Propanphosphonsäureanhydrid in Essigester zugegeben. Man rührte 1 h in der Kälte und über Nacht bei Raumtemperatur, wusch die organische Phase mit Wasser, 1 N NaOH und Wasser, trocknete über Na₂SO₄ und engte ein. Es verblieben 14,4 g (93,4 %) Produkt, Reinheit HPLC-Flächenprozent: 96,1 %. Der Gehalt an anderen Diastereomeren lag unter 1 %.

## Patentansprüche

1. Verfahren zur Herstellung von α-(N,N-Dialkylamino)-carbonsäureamiden der Formel I 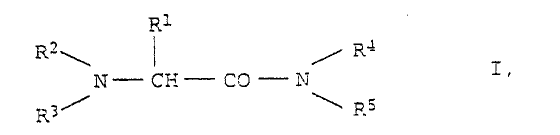 in der
R¹ C₁₋₆-Alkyl, C₃₋₇-Cycloalkyl, Phenyl, Benzyl, (CH₂)₃NH(C=NH)NH₂, CH₂CONH₂, CH₂CO₂H, CH₂SH, (CH₂)₂CONH₂, (CH₂)₂CO₂H, Imidazolyl-5-methylen, (CH₂)₄NH₂, (CH₂)₂SCH₃, CH₂OH, CH(OH)CH₃ oder Indolyl-β-methylen bedeutet, wobei reaktive Gruppen nach Bedarf mit Schutzgruppen versehen sind,
R² C₁₋₆-Alkyl oder ein mit C₁-C₄-Alkyl, C₁-C₄-Alkoxy, Nitro, CF₃, Cl, F oder Brom gegebenenfalls substituiertes Benzyl,
R³ C₁₋₆-Alkyl oder ein mit C₁-C₄-Alkyl, C₁-C₄-Alkoxy, Nitro, CF₃, Cl, F oder Brom gegebenenfalls substituiertes Eenzyl, wobei R¹ und R³ miteinander verknüpft sein können,
R⁴ und R⁵ unabhängig voneinander Wasserstoff, C₁₋₆-Alkyl, C₃₋₇-Cycloalkyl, Phenyl (welches durch 1, 2 oder 3 Fluor-, Chlor- oder Bromatome oder C₁₋₅-Alkyl-, C₁₋₅-Alkoxy- oder CF₃-Gruppen substituiert sein kann) einen aromatischen Heterocyclus (welcher durch 1, 2 oder 3 Fluor-, Chlor- oder Bromatome oder C₁₋₅-Alkyl-, C₁₋₅-Alkoxy- oder CF₃-Gruppen substituiert sein kann) oder Benzyl (welches durch 1, 2 oder 3 Fluor-, Chlor- oder Bromatome oder C₁₋₅-Alkyl-, C₁₋₅-Alkoxy- oder CF₃-Gruppen substituiert sein kann) bedeuten, wobei R⁴ und R⁵ noch zusätzlich miteinander verknüpft sein können, und in der NR⁴R⁵ auch für einen Aminosäure- oder einen Paptidrest stehen kann, wobei die Carboxylgruppe und andere funktionelle Gruppen eventuell mit Schutzgruppen blockiert sind,
**dadurch gekennzeichnet, daß** man die entsprechenden freien Säuren der Formel II 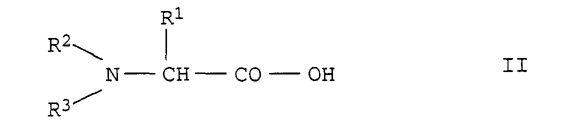 mit primären oder sekundären Aminen der Formel 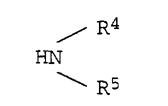 in Gegenwart von Anhydriden einer Alkanphosphonsäure umsetzt.

2. Verfahren nach Anspruch 1, **dadurch gekennzeichnet, daß** als primäre oder sekundäre Amine Aminosäurederivate oder Peptide eingesetzt werden.

3. Verfahren nach Anspruch 1, **dadurch gekennzeichnet, daß** man als Alkanphosphonsäureanhydrid n-Propanphosphonsäure anhydrid verwendet.

4. Verfahren nach Anspruch 1, **dadurch gekennzeichnet, daß** man als α-Dialkylaminocarbonsäuren N,N-Dimethyl- und N-Benzyl-N-methylaminocarbonsäuren einsetzt.

5. Verfahren nach Anspruch 1, **dadurch gekennzeichnet, daß** man als α-Dialkylaminocarbonsäuren N,N-Dimethylvalin, N,N-Dimethylisoleucin, N-Benzyl-N-methyl-valin oder N-Benzyl-N-methyl-isoleucin einsetzt.

## Claims

1. A process for preparing α-(N,N-dialkylamino) carboxamides of the formula I 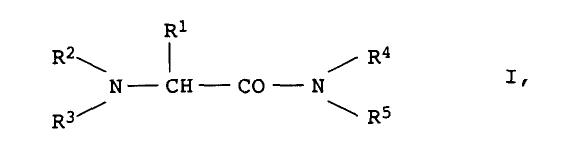 where
R¹ is C₁₋₆-alkyl, C₃₋₇-cycloalkyl, phenyl, benzyl, (CH₂)₃NH(C=NH)NH₂, CH₂CONH₂, CH₂CO₂H, CH₂SH, (CH₂)₂CONH₂, (CH₂)₂CO₂H, imidazolyl-5-methylene, (CH₂)₄NH₂, (CH₂)₂SCH₃, CH₂OH, CH(OH)CH₃ or indolyl-β-methylene, where reactive groups are, if required, provided with protective groups,
R² is C₁₋₆-alkyl or an unsubstituted or C₁-C₁-alkyl-, C₁-C₄-alkoxy, nitro-, CF₃-, Cl-, F- or bromine-substituted benzyl,
R³ is C₁₋₆-alkyl or an unsubstituted or C₁-C₁-alkyl-, C₁-C₄-alkoxy, nitro-, CF₃-, Cl-, F- or bromine-substituted benzyl, where R¹ and R³ can be linked together,
R⁴ and R⁵ are, independently of one another, hydrogen, C₁₋₆-alkyl, C₃₋₇-cycloalkyl, phenyl (which can be substituted by 1, 2 or 3 fluorine, chlorine or bromine atoms or C₁₋₅-alkyl, C₁₋₅-alkoxy or CF₃ groups), an aromatic heterocycle (which can be substituted by 1, 2 or 3 fluorine, chlorine or bromine atoms or C₁₋₅-alkyl, C₁₋₅-alkoxy or CF₃ groups) or benzyl (which can be substituted by 1, 2 or 3 fluorine, chlorine or bromine atoms or C₁₋₅-alkyl, C₁₋₅-alkoxy or CF₃ groups), where R⁴ and R⁵ can additionally be linked together, and where NR⁴R⁵ can also be an amino acid residue or peptide residue, the carboxyl group and other functional groups possibly being blocked with protective groups,
which comprises reacting the corresponding free acids of the formula II 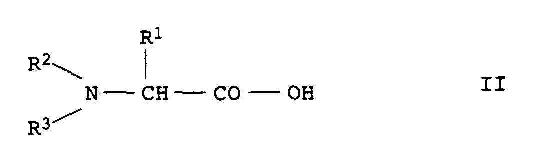 with primary or secondary amines of the formula 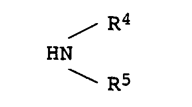 in the presence of anhydrides of an alkanephosphonic acid.

2. A process as claimed in claim 1, wherein amino acid derivatives or peptides are used as primary or secondary amines.

3. A process as claimed in claim 1, wherein n-propanephosphonic anhydride is used as alkanephosphonic anhydride.

4. A process as claimed in claim 1, wherein N,N-dimethyl- and N-benzyl-N-methylamino carboxylic acids are used as α-dialkylamino carboxylic acids.

5. A process as claimed in claim 1, wherein N,N-dimethylvaline, N,N-dimethylisoleucine, N-benzyl-N-methylvaline or N-benzyl-N-methylisoleucine is used as α-dialkylamino carboxylic acids.

## Revendications

1. Procédé pour la préparation d'alpha-(N,N-dialkylamino)carboxamides de formule I 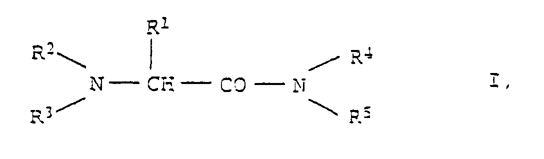 dans laquelle
R¹ représente un groupe alkyle en C1-C5, cycloalkyle en C3-C7, phényle, benzyle, (CH₂)₃NH(C=NH)NH₂, CH₂CONH₂, CH₂CO₂H, CH₂SH, (CH₂)₂CONH₂, (CH₂)₂CO₂H, imidazolyl-5-méthylène, (CH₂)₄NH₂, (CH₃)₂SCH₃, CH₂OH, CH(OH)CH₃ ou indolyl-bêta-méthylène, les groupes réactifs portant, en cas de besoin, des groupes protecteurs,
R² représente un groupe alkyle en C1-C5 ou un groupe benzyle portant éventuellement des substituants alkyle en C1-C4, alcoxy en C1-C4, nitro, CF₃, Cl, F ou Br,
R³ représente un groupe alkyle en C1-C6 ou un groupe benzyle portant éventuellement des substituants alkyle en C1-C4, alcoxy en C1-C4, nitro, CF₃, Cl, F ou Br, R¹ et R³ pouvant être reliés entre eux,
R⁴ et R⁵ représentent chacun, indépendamment l'un de l'autre, l'hydrogène, un groupe alkyle en C1-C5, cycloalkyle en C3-C7, phényle (lequel peut porter un, deux ou trois substituants fluoro, chloro, bromo ou alkyle en C1-C5, alcoxy en C1-C5 ou CF₃), un hétérocycle aromatique (lequel peut porter un, deux ou trois substituants fluoro, chloro ou bromo ou alkyle en C1-C5, alcoxy en C1-C5 ou CF₃) ou benzyle (lequel peut porter un, deux ou trois substituants fluoro, chloro ou bromo ou alkyle en C1-C5, alcoxy en C1-C5 ou CF₃), R⁴ et R⁵ pouvant en outre être reliés entre eux, NR⁴R⁵ pouvant également représenter un radical d'aminoacide ou de peptide, le groupe carboxyle et les autres groupes fonctionnels étant éventuellement bloqués par des groupes protecteurs,
**caractérisé par** le fait que l'on fait réagir les acides libres correspondants de formule II 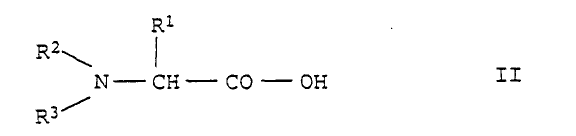 avec des amines primaires ou secondaires de formule 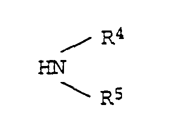 en présence d'anhydrides d'un acide alcanephosphonique.

2. Procédé selon la revendication 1, **caractérisé par** le fait que l'on utilise en tant qu'amines primaires ou secondaires des dérivés d'aminoacides ou des peptides.

3. Procédé selon la revendication 1, **caractérisé par** le fait que l'on utilise en tant qu'anhydride d'acide alcanephosphonique l'anhydride de l'acide n-propanephosphonique.

4. Procédé selon la revendication 1, **caractérisé par** le fait que les acides alpha-dialkylaminocarboxyliques mis en oeuvre sont des acides N,N-diméthyl- et N-benzyl-N-méthylaminocarboxyliques.

5. Procédé selon la revendication 1, **caractérisé par** le fait que les acides alpha-dialkylaminocarboxyliques mis en oeuvre sont la N,N-diméthylvaline, la N,N-diméthylisoleucine, la N-benzyl-N-méthylvaline ou la N-benzyl-N-méthyl-isoleucine.
